# EUROPEAN PATENT APPLICATION

(11) **EP 2 433 550 A1**
(43) Date of publication of application: **28.03.2012**
(21) Application number: 11181994.2
(22) Date of filing: 20.09.2011
(51) Int. Cl.: A61B 1/00, A61B 1/015, A61M 1/00, A61M 39/00

(54) **Suction button assembly for endoscope**

(30) Priority: 22.09.2010 JP 2010211857
(71) Applicant: FUJIFILM Corporation, Tokyo 106-0031 (JP)
(72) Inventor: YAMANE, Kenji, Kanagawa (JP)
(74) Representative: Klunker . Schmitt-Nilson . Hirsch

(57) **Abstract**

A suction button assembly of a valve structure for an endoscope includes a cylinder housing (30), cylinder passage (40-42), plunger (25) or piston unit, cylinder opening (32), plunger stem, flow opening (45), valve sleeve (53) and flow channel (60). A valve opening is formed in a side surface of the valve sleeve at an end of the flow channel, closed by a wall of the cylinder passage when the plunger is in the closed position, for shut-off between the suction channel (17) and the discharge conduit (21), and registered with the flow opening when the plunger is in the open position, for communication between the suction channel and the discharge conduit. A valve engaging surface (42) is formed partially with the cylinder passage, has the flow opening, and has an inner diameter decreasing in an upward direction. The valve sleeve is tapered and tightly contacted on the valve engaging surface when the plunger is in the closed position.

## Description

### BACKGROUND OF THE INVENTION

### 1. Field of the Invention

The present invention relates to a suction button assembly for an endoscope. More particularly, the present invention relates to a suction button assembly for an endoscope, in which resistance between a plunger and a cylinder housing can be small, and suction of fluid from a body cavity can be controlled precisely.

### 2. Description Related to the Prior Art

An endoscope includes an operation unit and an elongated tube extending distally from the operation unit. The elongated tube includes a suction channel and a distal opening. The suction channel extends in an axial direction. The distal opening is formed in a distal end face of the elongated tube, as a distal end of the suction channel. A suction button assembly is incorporated in the operation unit. A specific example of the suction channel is an instrument channel, which is used for penetration of a forceps or other medical instrument and for supply of washing water or the like. A portion of the suction channel is a branch of the instrument channel, and is connected to the suction button assembly.

In addition to the suction channel, a discharge conduit for suction toward a negative pressure source is connected to the suction button assembly. An example of the negative pressure source is a suction pump. The suction button assembly, when depressed manually by a doctor or operator, connects the discharge conduit to the suction channel for carry out suction through the distal opening. The suction button assembly, when left without the depression, disconnects the discharge conduit from the suction channel to discontinue the suction.

JP-A 2008-228990, JP-A 2007-252589 and JP-A 2007-185276 disclose examples of the suction button assembly. In Fig. 10, the suction button assembly includes a cylinder housing 100, a cylinder passage 101 with an inner wall, a plunger 102 or piston unit, and a cap device 103. To a suction channel 99 in the operation unit, a lower end of the cylinder housing 100 is connected. The cylinder passage 101 is formed through the cylinder housing 100. The plunger 102 is contained in the cylinder passage 101. The cap device 103 couples an upper end of the plunger 102 to an upper end of the cylinder housing 100.

A flow opening 105 for a negative pressure source is formed in an intermediate portion of the cylinder passage 101. A negative pressure source 104 has a conduit connected to the flow opening 105. A valve engaging surface 106 is disposed in the cylinder passage 101. The plunger 102 includes a plunger stem 102a and a valve head 102b. The plunger stem 102a is slidable in the cylinder passage 101. The valve head 102b is formed with a lower end of the plunger stem 102a. A cylinder opening 107 is formed at an upper end of the cylinder housing 100. A stem end of the plunger 102 is disposed to protrude from the cylinder opening 107. A passage channel 108 is formed in an outer wall of the plunger stem 102a and extends in the longitudinal direction. The cap device 103 is formed from resilient material such as rubber, and biases the plunger end of the plunger 102 to protrude from the cylinder opening 107.

When the stem end of the plunger 102 is left without depression, the valve head 102b contacts the valve engaging surface 106 for shut-off between the negative pressure source 104 and the suction channel 99. When the stem end of the plunger 102 is depressed against the bias of the cap device 103 into the cylinder opening 107 at a predetermined shift, the valve head 102b moves away from the valve engaging surface 106 for communication between the negative pressure source 104 and the suction channel 99. Fluid such as body fluid and mixed particles, drawn from the suction opening, is transferred from the suction channel 99 between the plunger 102 and the cylinder passage 101 and through the passage channel 108 as indicated with the phantom line. Then the fluid is caused to flow through the passage channel 108 to the negative pressure source 104. In the course of this, particles of a considerably large size cannot be passed through the cylinder passage 101, because the passage channel 108 and a clearance space are too small.

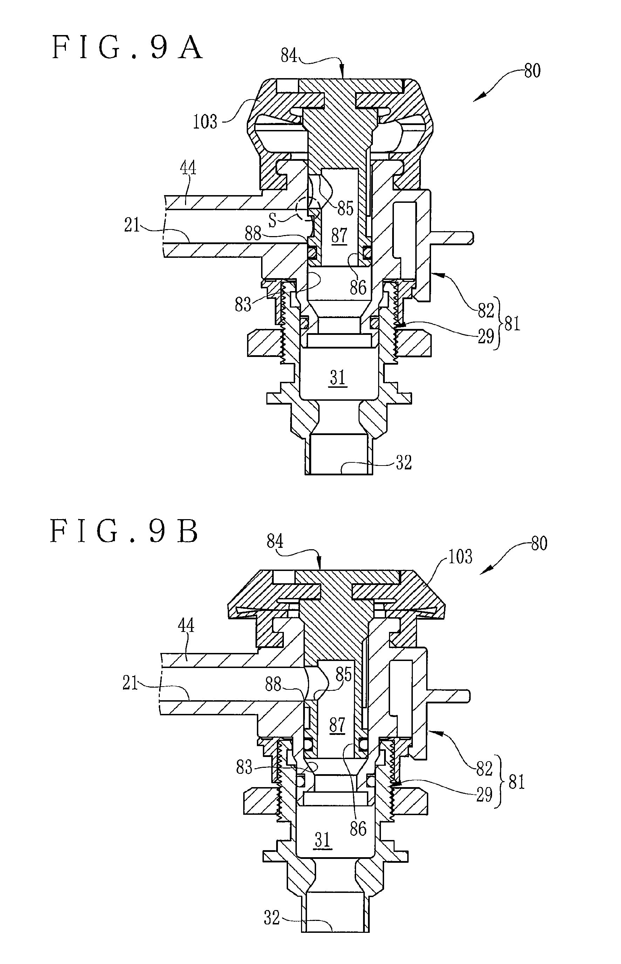

JP-A 2004-166944 discloses a suction button assembly, in which a plunger of a long shape has a flow channel for flow of fluid such as body fluid and mixed solid particles. A valve opening on a lateral side is formed on a lateral side of the plunger. A lower opening is formed in a lower end of a lower stem end. The flow channel extends from the valve opening to the lower opening. When an upper stem end of the plunger is depressed at a predetermined shift by manual operation, then a flow opening for the negative pressure source becomes opposed to the valve opening. See Fig. 9B. The fluid drawn from the distal opening flows through the flow channel to a discharge conduit. The flow channel may be constructed to have a larger diameter than the above-mentioned clearance space, so that mixed solid particles with a relatively large diameter can be removed as fluid.

The suction button assembly according to JP-A 2004-166944 is unlike JP-A 2008-228990, JP-A 2007-252589 and JP-A 2007-185276 having the valve head at the lower stem end of the plunger. In the document, a seal such as O-ring is disposed on the plunger nearer to the lower stem end than the valve opening. While the upper stem end in the plunger is left without depression and if the valve opening is offset from the flow opening upwards in the plunger longitudinal direction, then the O-ring operates for shut-off between the flow opening and the valve opening.

In the suction button assembly of JP-A 2004-166944, the O-ring is fitted on the plunger. When the plunger is slid in the cylinder, friction occurs in contact of the O-ring with an inner wall of the flow channel. Resistance of the friction may be so large that failure of the plunger will occur seriously, for example the plunger will fail to return to its original position even though the cap device biases upon leaving the plunger without the depression.

### SUMMARY OF THE INVENTION

In view of the foregoing problems, an object of the present invention is to provide a suction button assembly for an endoscope, in which resistance between a plunger and a cylinder housing can be small, and suction of fluid from a body cavity can be controlled precisely.

In order to achieve the above and other objects and advantages of this invention, a suction button assembly for an endoscope is provided, the endoscope including an operation unit, a section of an elongated tube for entry in a body cavity, and a suction channel and a discharge conduit disposed with the operation unit, wherein a distal opening is formed in a distal end of the elongated tube, the suction channel is disposed to extend to the distal opening, the discharge conduit is connected to a negative pressure source. In the suction button assembly, a cylinder housing is disposed on the operation unit, and includes a cylinder passage, a valve engaging surface and a flow opening, the cylinder passage being formed through the cylinder housing, the valve engaging surface being disposed in the cylinder passage, the flow opening formed in a wall of the cylinder passage, the cylinder passage having first and second cylinder openings, the first cylinder opening being connected to the suction channel, the valve engaging surface being tapered with an inner diameter decreasing from the first cylinder opening toward the second cylinder opening, the discharge conduit extending from the flow opening. A plunger is contained in the cylinder passage in a slidable manner, and includes a stem end, a valve head and a flow channel, the stem end protruding from the second cylinder opening, the valve head being movable to and from the valve engaging surface for contact, the flow channel being opposed to the flow opening, wherein the plunger is in a closed position when the stem end is free without being depressed, and is in an open position when the stem end is depressed, and the valve head, when the plunger is in the closed position, contacts the valve engaging surface for interruption between the suction channel and the discharge conduit, and when the plunger is in the open position, leaves from the valve engaging surface for communication between the suction channel and the discharge conduit in cooperation with the flow channel aligned with the flow opening.

Preferably, the cylinder passage further includes a lower inner wall, disposed between the valve engaging surface and the first cylinder opening, and connected in series with the suction channel. An upper inner wall is disposed between the valve engaging surface and the second cylinder opening, having a smaller inner diameter than the lower inner wall. A maximum outer diameter of the valve head is smaller than an inner diameter of the lower inner wall, and the upper inner wall contains the stem end in a slidable manner.

Preferably, furthermore, a biasing device biases the plunger toward the closed position. The plunger in the closed position is set in the open position by depressing the stem end into the cylinder passage against the biasing device.

Preferably, the biasing device is a cap device, secured to the stem end and the cylinder housing, disposed to cover a periphery of the stem end, and formed from resilient material.

Preferably, the flow channel is a channel, formed through the plunger, and having a flow port and a valve opening, the flow port being formed in a lower stem end of the plunger opposite to the stem end, the valve opening being formed in a wall of the plunger. The valve opening is positioned in the cylinder passage, and opposed to the flow opening when the plunger is in the open position.

Preferably, the valve head is disposed at the lower stem end. The flow channel has an inner diameter increasing toward the flow port in the valve head.

Preferably, furthermore, a regulating device for preventing the plunger from rotating about an axis of the cylinder housing, to keep the valve opening aligned with the flow opening.

Also, a suction button assembly for an endoscope is provided, the endoscope including an operation unit, a section of an elongated tube, disposed to extend from the operation unit, for entry in a body cavity, a distal opening formed at a distal end of the elongated tube, a suction channel formed in the operation unit to extend through the elongated tube toward the distal opening, a discharge conduit, formed with the operation unit, and connected to a negative pressure source, the suction button assembly having a valve structure for changing over communication and shut-off between the suction channel and the discharge conduit. A cylinder housing is secured to the operation unit. A cylinder passage is formed within the cylinder housing to extend vertically, and has a lower end connected with the suction channel. A flow opening is formed in an inner wall of the cylinder passage, and disposed at one end of the discharge conduit. A plunger is contained in the cylinder passage in a slidable manner between open and closed positions, for opening and closing the flow opening. A tapered valve engaging surface is disposed at a portion of the cylinder passage, has an inner diameter increasing in a downward direction, and has the flow opening. A tapered valve head is formed with the plunger, has an outer diameter increasing in the downward direction, for reception on the valve engaging surface in tight contact therewith when the plunger is in the closed position, and for separation from the valve engaging surface when the plunger is in the open position. A valve opening is formed in a side wall of the valve head, closed by the inner wall of the cylinder passage when the plunger is in the closed position, for shut-off between the suction channel and the discharge conduit, and registered with the flow opening when the plunger is in the open position, for communication between the suction channel and the discharge conduit.

Preferably, furthermore, an upper inner wall is disposed at a portion of the cylinder passage to extend upwards from the valve engaging surface. A plunger stem is disposed in the plunger to extend from the valve head upwards, and contained in the upper inner wall in a slidable manner. A cylinder opening is formed at an upper end of the cylinder passage, wherein a stem end of the plunger stem protrudes through the cylinder opening.

Preferably, furthermore, a lower inner wall is disposed at a portion of the cylinder passage to extend from a lower end of the valve engaging surface toward the suction channel. A maximum outer diameter of the valve head is smaller than an inner diameter of the lower inner wall.

Preferably, furthermore, a biasing device biases the plunger to the closed position. The plunger in the closed position is shifted to the open position by depressing the stem end through the cylinder opening against the biasing device.

Preferably, the biasing device is a cap device of a resilient material, and includes a cap top portion of a disk shape connected with the stem end. A resilient cap skirt is disposed to project in the downward direction from an edge of the cap top portion, has a lower end secured to the cylinder housing, is compressed in the downward direction by depressing force of the plunger toward the open position, and extended by return force thereof upon release of the plunger from the depressing force, for returning the plunger to the closed position.

Preferably, furthermore, a cylinder end surface is disposed on the cylinder housing and opposed to the cap device. A lower wall is disposed under the cap device, received by the cylinder end surface when the plunger stem is slid in the downward direction, for preventing the plunger stem from sliding beyond a predetermined shift.

Preferably, the discharge conduit extends in a direction perpendicular to sliding of the plunger.

Preferably, furthermore, a regulating device prevents the plunger in the open position from rotating on an axis of the cylinder housing, to oppose the valve opening to the flow opening.

Preferably, furthermore, a vent channel is formed in a wall of the discharge conduit, for venting of the discharge conduit. A closure device is secured to the cylinder housing, for closing the vent channel when the plunger is in the open position, and for opening the vent channel when the plunger is in the closed position.

Preferably, furthermore, a cap device is coupled to the stem end and to an upper end of the cylinder housing, for covering the plunger over the cylinder housing. A vent hole is formed with the vent channel to open externally, and opposed to the cap device. The closure device has a closure surface, formed under the cap device, for covering the vent hole when the plunger is in the open position, and for uncovering the vent hole with an inclination when the plunger is in the closed position.

Preferably, an inner diameter of a valve sleeve of the valve head increases in the downward direction in a form according to the valve engaging surface.

Preferably, the negative pressure source is a suction pump.

Preferably, the operation unit includes a handle housing for containing the suction channel partially. Furthermore, a support sleeve is secured to the handle housing, for supporting the cylinder housing, and for connecting the cylinder passage to the suction channel.

Preferably, furthermore, a through opening is formed in the cap device, positioned over the cylinder opening, for receiving entry of the stem end. A receiving recess is formed in the cap device at an upper end of the through opening, and has a larger inner width than the through opening. A shaft head is formed with the stem end to project radially from a periphery thereof, fitted in the receiving recess, for securing the plunger to the cap device.

Therefore, resistance between a plunger and a cylinder housing can be small, because the structure including the valve engaging surface and the tapered valve head is used.

### BRIEF DESCRIPTION OF THE DRAWINGS

The above objects and advantages of the present invention will become more apparent from the following detailed description when read in connection with the accompanying drawings, in which:
Fig. 1 is a plan illustrating an endoscope;
Fig. 2 is a vertical section illustrating a suction button assembly of the endoscope;
Fig. 3 is a vertical section illustrating the suction button assembly at the time of depression;
Fig. 4A is a vertical section illustrating a cylinder housing;
Fig. 4B is a perspective view illustrating the cylinder housing;
Fig. 5A is a perspective view illustrating a plunger;
Fig. 5B is a rear perspective view illustrating the plunger;
Fig. 6 is a vertical section illustrating a sliding valve sleeve in enlargement;
Fig. 7 is a perspective view, partially broken illustrating a cap device;
Fig. 8A is a vertical section illustrating a closed state with the sliding valve sleeve;
Fig. 8B is a vertical section illustrating an open state of a seat opening;
Fig. 9A is a vertical section illustrating a suction button assembly of a known structure;
Fig. 9B is a vertical section illustrating the suction button assembly of the known structure at the time of depression;
Fig. 10 is a vertical section illustrating a suction button assembly of another known structure having a valve head and a valve engaging surface.

### DETAILED DESCRIPTION OF THE PREFERRED EMBODIMENT(S) OF THE PRESENT INVENTION

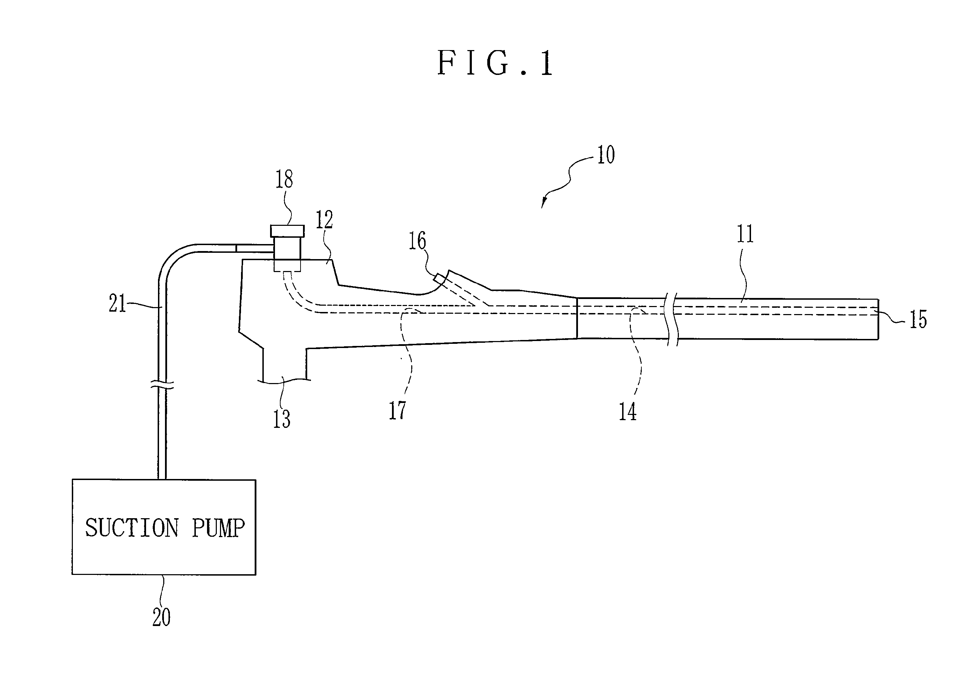

In Fig. 1, an endoscope 10 is a bronchoscope, and includes an elongated tube 11 or guide tube, an operation unit 12 and a universal cable 13. The elongated tube 11 is entered in a bronchus as a body cavity. The operation unit 12 is disposed at a proximal end of the elongated tube 11. An endoscope system for use includes a processing apparatus (not shown) and a light source apparatus (not shown). The universal cable 13 is connected to those apparatuses.

An instrument channel 14 is formed in the elongated tube 11 for entry of a forceps or other instrument for treatment. A distal instrument opening 15 is disposed at a distal end of the instrument channel 14, and open in a distal surface of the elongated tube 11. A proximal instrument opening 16 is disposed at a proximal end of the instrument channel 14, and open in the operation unit 12. A seal cap (not shown) is fitted in the proximal instrument opening 16 for closing at a normal time before entry of the instrument. Note that a syringe (not shown) can be connected with the proximal instrument opening 16 for injecting washing water such as physiological saline water. The water flows through the instrument channel 14 and is ejected by the distal instrument opening 15.

Windows (not shown) are formed in the distal surface of the elongated tube 11 in addition to the distal instrument opening 15, including an imaging window and lighting window. An image pickup device (not shown) is disposed behind the imaging window. An optical fiber cable (not shown) is disposed to extend from the lighting window. Signal lines and the optical fiber cable for the image pickup device are disposed to extend through the elongated tube 11 and the universal cable 13, and connected to the processing apparatus and light source apparatus.

The instrument channel 14 is used for transfer of fluid from the distal instrument opening 15 by suction, such as blood, waste fluid with mixed solid particles, or other body fluid. A suction channel 17 is formed in the operation unit 12 and extends as a branch of the instrument channel 14. A suction button assembly 18 or shut-off valve assembly of the invention is incorporated in the operation unit 12. The suction channel 17 extends to the suction button assembly 18.

A negative pressure source 20 or suction pump is installed externally. A discharge conduit 21 of the operation unit 12 is a flow line from the suction button assembly 18 to the negative pressure source 20. When the suction button assembly 18 is depressed or left without depression, a path is changed over for opening and closing between the suction channel 17 and the discharge conduit 21. The negative pressure source 20 is always used for suction during the imaging with the endoscope.

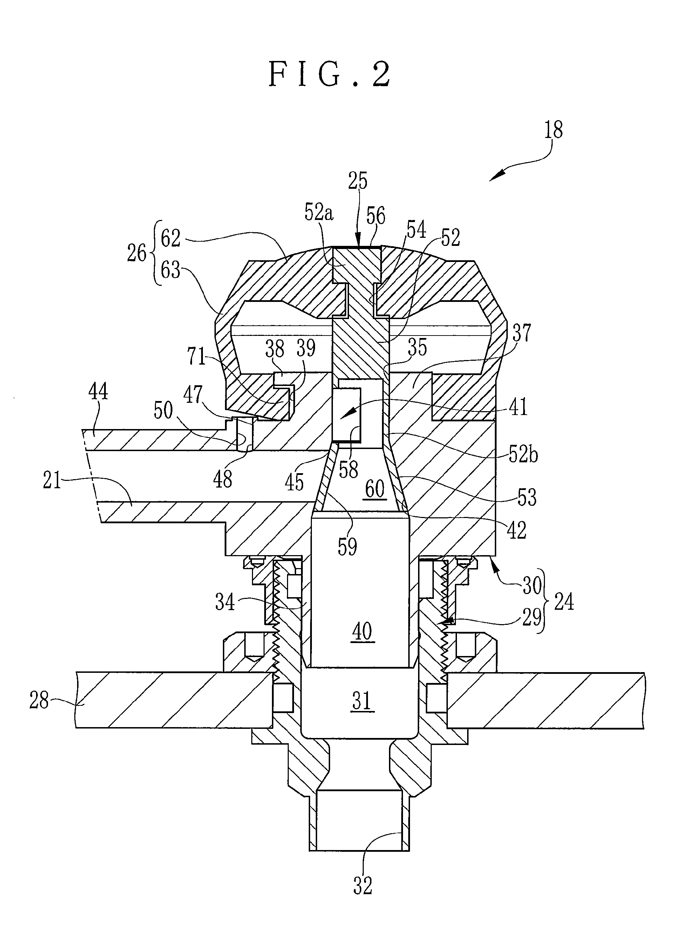

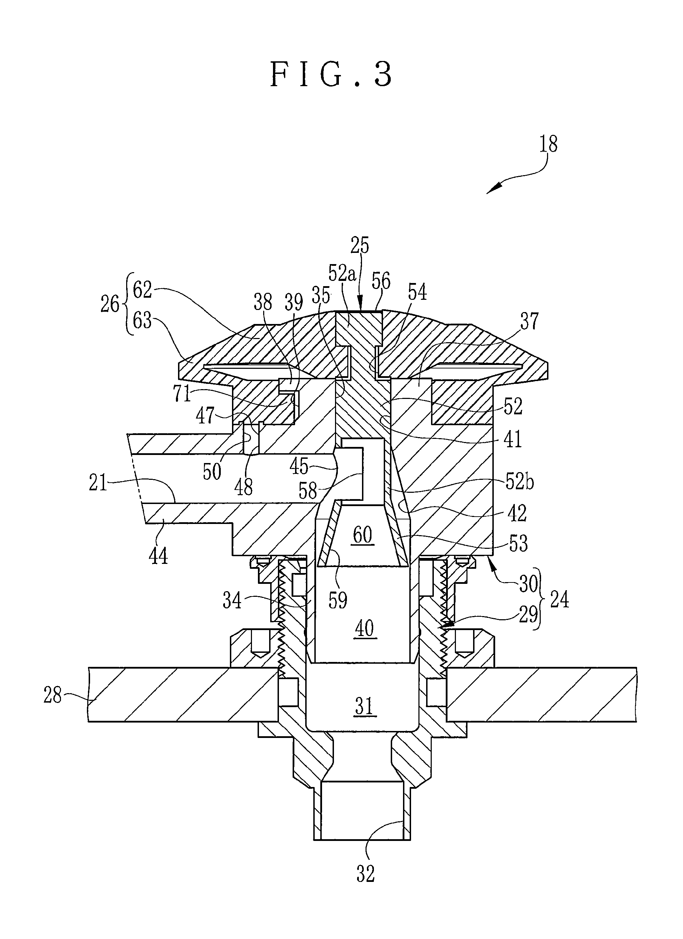

In Fig. 2, an initial state without depression is illustrated. In Fig. 3, a depressed state is illustrated. The suction button assembly 18 includes a housing device 24 or cylinder device, a plunger 25 or piston unit, and a cap device 26. The housing device 24 is attached to the operation unit 12. The plunger 25 is contained in the housing device 24. The cap device 26 connects the plunger 25 to the housing device 24 and covers those.

The operation unit 12 has a handle housing 28. The housing device 24 includes a support sleeve 29 or valve housing, and a cylinder housing 30 or valve guide. The support sleeve 29 is fixedly secured to the handle housing 28. The cylinder housing 30 is fixedly connected with the support sleeve 29. Note that an upper side in the drawings is referred to as an output side or upper side, a lower side being referred to as an input side.

The support sleeve 29 has an output end protruding to the outside of the handle housing 28, and an input end protruding to the inside of the handle housing 28. The support sleeve 29 is fixedly secured to the handle housing 28. A flow chamber 31 in the support sleeve 29 extends in the longitudinal direction. A valve port 32 for suction (lower cylinder end) is formed at an input end of the support sleeve 29 for connection with the suction channel 17. The flow chamber 31 communicates with the suction channel 17 by means of the valve port 32. An output end of the flow chamber 31 is connected with the cylinder housing 30.

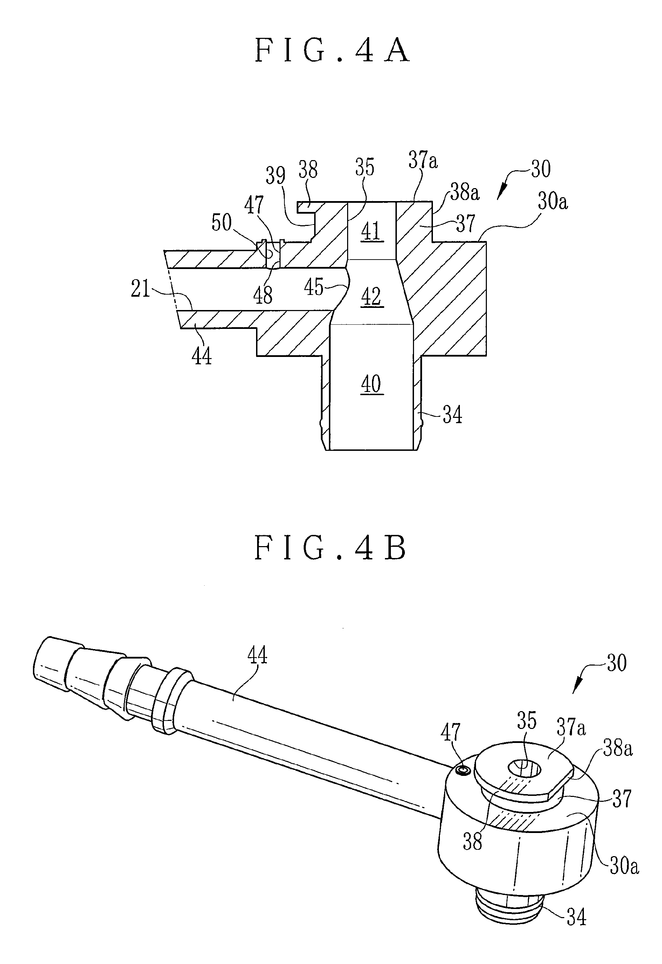

A flow sleeve 34 is a portion at an input end of the cylinder housing 30. An output end of the support sleeve 29 is connected with the flow sleeve 34, which is concentric with the flow chamber 31. A cylinder opening 35 is formed in an upper cylinder end of the cylinder housing 30, and causes an upper end of the plunger 25 to protrude. See Figs. 4A and 4B.

An end surface 30a is disposed at an end of the housing device 24 of Figs. 4A and 4B. A cylinder sleeve 37 is disposed to project from the end surface 30a for connection with the cap device 26. In Figs. 4A and 4B, an upper cylinder surface 37a is disposed at an end of the cylinder sleeve 37. The cylinder opening 35 is open in the upper cylinder surface 37a. A bonnet flange 38 projects from an outer surface of the cylinder sleeve 37. In Fig. 4B, a cap regulating surface 38a in a regulating device is formed in the bonnet flange 38 by chamfering flatly, and prevents the cap device 26 from rotating. In Fig. 4A, a sealing groove 39 is defined annularly by plural surfaces including a lower surface of the bonnet flange 38, the outer surface of the cylinder sleeve 37 and the end surface 30a, for connection with the cap device 26.

In Fig. 4A, the cylinder housing 30 includes a cylinder passage, which is constituted by a first inner wall 40, a second inner wall 41 (cylinder bores), and a valve engaging surface 42 tapered conically or third inner wall or inner tapered wall. The first inner wall 40 extends in parallel with the flow chamber 31, and is connected to the suction channel 17 by the flow chamber 31. The second inner wall 41 is disposed at an upper side, extends coaxially with the first inner wall 40, has a smaller inner width than the first inner wall 40. An upper end of the second inner wall 41 has the cylinder opening 35. The valve engaging surface 42 is shaped with a decreasing inner diameter, and extends from the output end of the first inner wall 40 to a lower end of the second inner wall 41.

In Fig. 4B, a valve port sleeve 44 is included in the cylinder housing 30, positioned beside the valve engaging surface 42, and connected to the discharge conduit 21. The valve port sleeve 44 extends in a direction perpendicular to each of the inner walls 40 and 41 and in series with the discharge conduit 21. A flow opening 45 or seat opening for a negative pressure source is formed in the valve engaging surface 42, and at an end of the valve port sleeve 44.

A vent channel 50 is formed through the cylinder housing 30. A vent hole 47 is an outer open end of the vent channel 50 and opposed to the cap device 26. An inner hole 48 is an inner open end of the vent channel 50 and positioned inside the valve port sleeve 44. The discharge conduit 21 is open to the atmosphere through the vent channel 50.

In Figs. 2 and 3, the plunger 25 is contained in the cylinder housing 30 slidably in a longitudinal direction, and switches the open and closed states between the suction channel 17 and the discharge conduit 21 upon operation for depressing and leaving in a non-depressed state. The plunger 25 is one piece including a plunger stem 52 or piston rod, and a sliding tapered valve sleeve 53 or valve head. The plunger stem 52 extends in a longitudinal direction of the inner walls 40 and 41 and the valve engaging surface 42. The plunger stem 52 is mounted in the second inner wall 41 slidably in the longitudinal direction. A first stem end 52a of the plunger stem 52 protrudes from the cylinder opening 35.

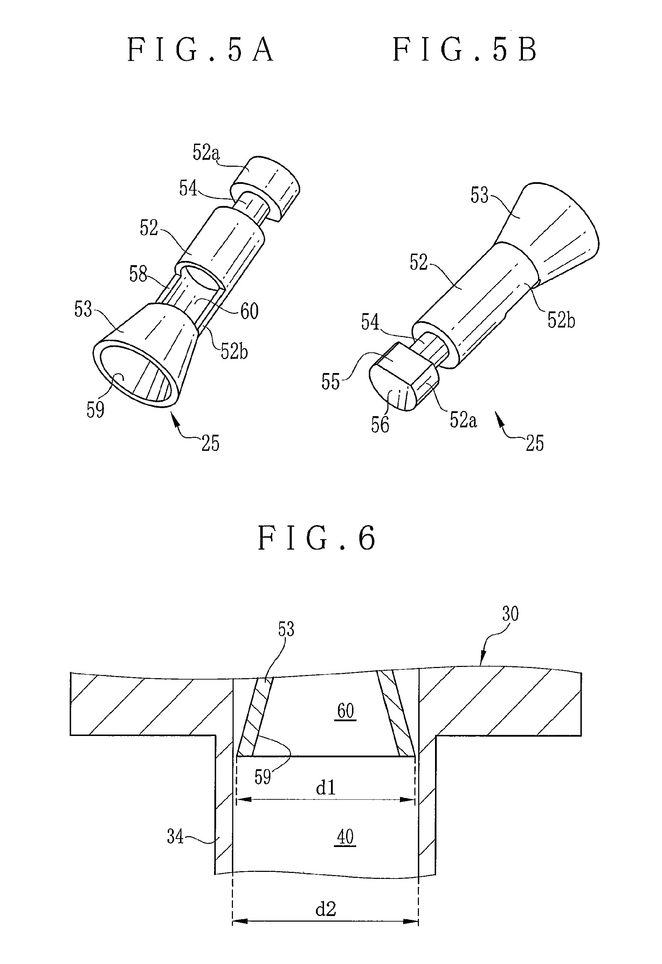

In Figs. 5A and 5B, a cap sealing groove 54 is formed in the periphery of the first stem end 52a annularly, and used for connecting the cap device 26. A regulating surface 55 in a regulating device of Fig. 5B is formed with the first stem end 52a, is disposed higher than the cap sealing groove 54, has a flatly chamfered shape, and prevents the plunger 25 from rotating accidentally in the cap device 26. A shaft head 56 for depression is disposed on the first stem end 52a, and pushed manually for depression.

At a second stem end 52b of the plunger stem 52, the tapered valve sleeve 53 is disposed. An outer valve head of the tapered valve sleeve 53 extends along the valve engaging surface 42. The tapered valve sleeve 53 slides through the second inner wall 41 and the valve engaging surface 42 upon sliding of the plunger stem 52.

The plunger 25, when left without the depression, closes the flow opening 45 by contact of the tapered valve sleeve 53 with the valve engaging surface 42. A closed state is defined by shut-off between the suction channel 17 and the discharge conduit 21. The plunger 25, when the first stem end 52a is pressed into the cylinder opening 35 with a predetermined shift by the depression, opens the flow opening 45 by setting the tapered valve sleeve 53 away from the valve engaging surface 42. An open state is defined to communicate between the suction channel 17 and the discharge conduit 21. Note that the predetermined shift is defined by a position short of disabling further depression because of engagement of the cap device 26 with the upper cylinder surface 37a.

In Figs. 5A and 5B, the plunger 25 has a valve opening 58, a flow port 59 and a flow channel 60. The valve opening 58 is formed in a side wall of the plunger stem 52. The flow port 59 is open downwards in the tapered valve sleeve 53. The flow channel 60 extends between the valve opening 58 and the flow port 59. The valve opening 58 is open in the side wall of a portion of the plunger stem 52 opposed to the flow opening 45 when the plunger 25 is in the open state. The flow channel 60, when the plunger 25 is in the open state, causes fluid in the flow port 59 to flow toward the valve opening 58, such as body fluid and mixed solid particles. A diameter of the flow channel 60 is constant in the plunger stem 52, but increases gradually in the tapered valve sleeve 53 toward the flow port 59.

In Fig. 6, a maximum outer diameter d1 of the tapered valve sleeve 53 is set smaller than an inner diameter d2 of the first inner wall 40. This prevents the tapered valve sleeve 53 from frictionally contacting the first inner wall 40 when the plunger 25 is slid in the cylinder housing 30. Note that the plunger stem 52 has a diameter slightly smaller than an inner diameter of the second inner wall 41, and is guided by the second inner wall 41.

In Figs. 2 and 3, the cap device 26 is formed from elastic material such as rubber and elastomer. The cap device 26 includes a circular cap top portion 62 or cap head, and a cap skirt 63. The cap top portion 62 is coupled to the first stem end 52a. The cap skirt 63 is coupled to the distal end of the cylinder housing 30.

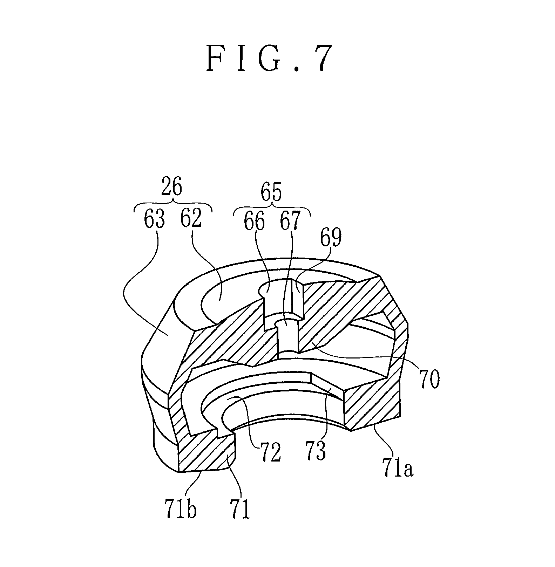

In Fig. 7, a through opening 65 is formed in the cap top portion 62 for receiving entry of the plunger 25. The through opening 65 has a receiving recess 66 and a seal hole 67 or entry hole. The receiving recess 66 is formed in an upper surface of the cap top portion 62, and receives entry of an upper part of the first stem end 52a higher than the cap sealing groove 54. The seal hole 67 receives entry of a portion of the plunger stem 52 having the cap sealing groove 54.

A regulating surface 69 in a regulating device is formed flatly by chamfering a side wall of the receiving recess 66, and firmly engaged with the regulating surface 55 of the first stem end 52a. This prevents the plunger 25 from rotating in the cap device 26. A lower hole wall 70 or first ring in the cap top portion 62 is defined by the seal hole 67, and is fitted in the cap sealing groove 54 of the first stem end 52a. Thus, the first stem end 52a is connected to the cap top portion 62.

An upper end of the cap skirt 63 is flush with the cap top portion 62. An annular ridge 71 or second ring is formed at a lower end of the cap skirt 63, and projects in an inward direction. The annular ridge 71 is fitted in the sealing groove 39. Thus, the cap device 26 is retained on the cylinder housing 30. A mating surface 71a outside the annular ridge 71 is opposed to the end surface 30a. A portion of the mating surface 71a opposed to the vent hole 47 is a beveled closure surface 71b or closure device, which extends with an increasing distance from the end surface 30a in a radial direction of the plunger 25. The closure surface 71b operates for control of venting according to the invention.

A flange receiving recess 72 is formed in an upper wall of the annular ridge 71 around the opening, and is engaged with the bonnet flange 38. A seat positioning surface 73 in a regulating device is formed with the an inner wall of the flange receiving recess 72, has a flat shape, and is firmly engaged with the cap regulating surface 38a of the bonnet flange 38. This prevents the cap device 26 from rotating about the axis of the cylinder housing 30. Also, the plunger 25 is prevented from rotating in the cylinder housing 30 indirectly by the cap device 26. It is possible to oppose the valve opening 58 to the flow opening 45 when the plunger 25 is set in the open position.

In Figs. 2 and 3, return force of the cap device 26 biases the plunger 25 upwards through the cylinder opening 35, to maintain the plunger 25 in the closed state. When the first stem end 52a is pressed into the cylinder opening 35, the cap top portion 62 of the cap device 26 moves toward the cylinder opening 35. In response to this, the cap skirt 63 deforms resiliently, in other words, becomes larger radially around the plunger 25 and becomes smaller (compressed) in the plunger longitudinal direction. When the suction button assembly is left without the depression, the cap device 26 returns to its original shape owing to the return force of the cap skirt 63.

At the time of the compression by the deformation, the annular ridge 71 of the cap skirt 63 is pressed toward the end surface 30a. Thus, the vent channel 50 is closed by contact of the closure surface 71b with the vent hole 47. See Fig. 3. When the cap skirt 63 returns to its original shape, the closure surface 71b comes away from the vent hole 47, to open the discharge conduit 21 to the atmosphere by uncovering the vent channel 50. See Fig. 2.

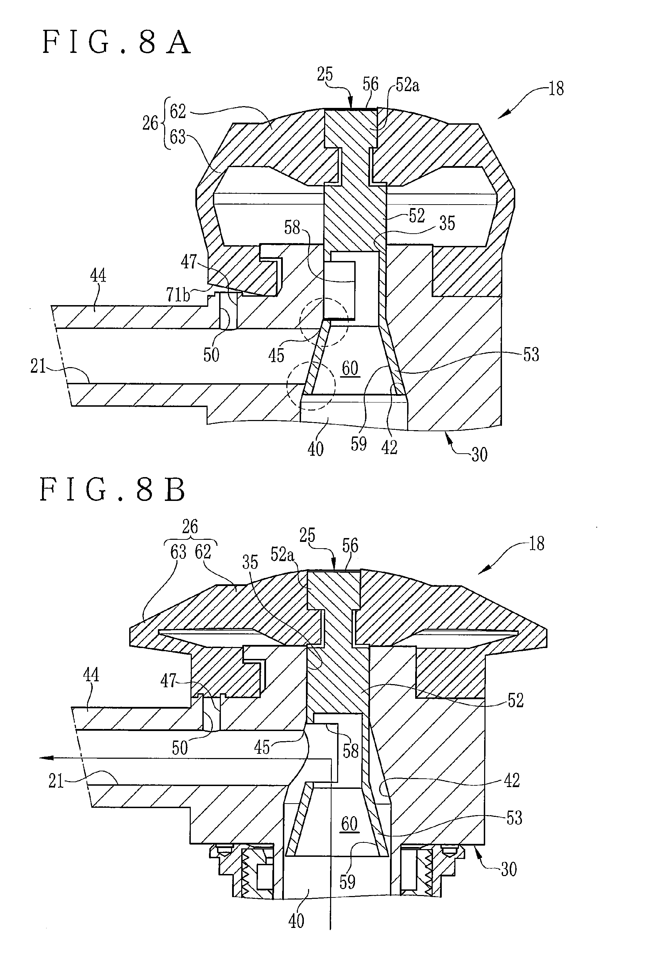

The operation of the suction button assembly 18 is described now. For diagnosis through the endoscope, the negative pressure source 20 is always used for suction. In Fig. 8A, an initial state without the suction is illustrated. The plunger 25 or piston unit is set in the closed state with the cap device 26, to press the outer valve head of the tapered valve sleeve 53 on the valve engaging surface 42 or inner tapered wall. The valve head of the tapered valve sleeve 53 tightly contacts the periphery of the flow opening 45 or seat opening, because shaped for suitable contact with the valve engaging surface 42 (indicated by the broken lines).

As the outer and inner walls are conical specifically, one of portions of the outer wall can close the flow opening 45 even when there is an error in the position or shape of the flow opening 45 or irregularity in the position of the plunger 25. The flow opening 45 can be closed tightly without a clearance space reliably. It is possible to discontinue the suction in the distal instrument opening 15 by shut-off between the suction channel 17 and the discharge conduit 21, even without attaching a seal such as an O-ring around the plunger 25 or near to the flow opening 45.

When the plunger 25 is in the closed position, the closure surface 71b of the cap skirt 63 is away from the vent hole 47 with a clearance space. The vent channel 50 is open. Thus, the discharge conduit 21 is open to the atmosphere. It is possible to prevent occurrence of overload to the negative pressure source 20 even when the flow opening 45 is closed by the tapered valve sleeve 53.

For the suction, the shaft head 56 is depressed as illustrated in Fig. 8B. The first stem end 52a is pushed into the cylinder opening 35 against the resiliency of the cap device 26. The cap skirt 63 of the cap device 26 is resiliently deformed to enlarge the diameter and compress vertically, so that the cap top portion 62 contacts the upper cylinder surface 37a. Thus, the plunger 25 is switched from the closed state to the open state. The tapered valve sleeve 53 shifts away from the valve engaging surface 42 to open the flow opening 45.

When the cap skirt 63 is deformed resiliently, the vent channel 50 is closed by tight contact of the closure surface 71b with the vent hole 47. A negative pressure or force of suction in the discharge conduit 21 increases because of its separation from the atmosphere.

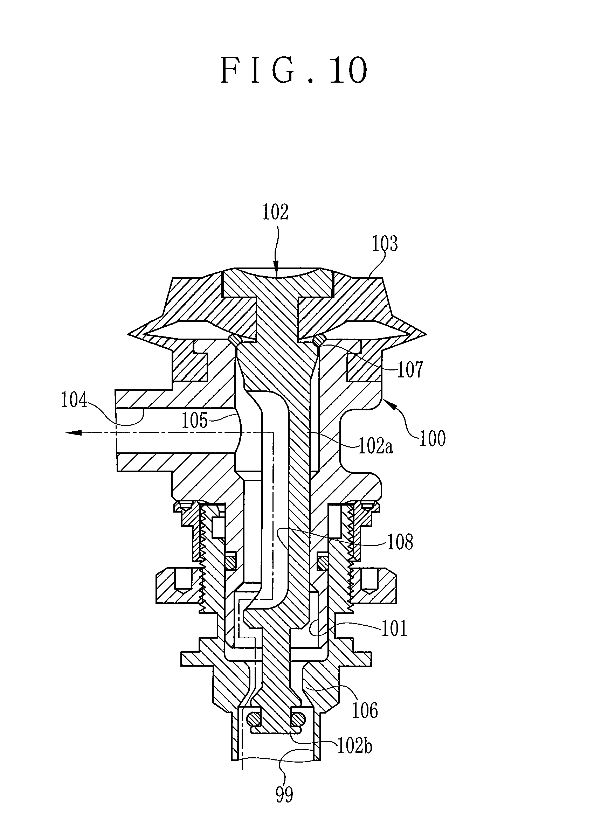

When the flow opening 45 is open, the suction channel 17 becomes connected with the discharge conduit 21, to suck fluid through the distal instrument opening 15, such as body fluids, mixed solid particles and the like. The fluid flows through the suction channel 17, the valve port 32 and the flow chamber 31, is drawn into the first inner wall 40 of the cylinder housing 30 as indicated by the arrow, and enters the flow opening 45 upon passing the flow channel 60. The flow channel 60 is shaped with a considerably large diameter in the plunger 25 without forming a path for fluid between an inner wall of the cylinder housing 30 and an outer wall of the plunger 25 according to the known example in Fig. 10. A flow channel width in the housing device 24 where fluid can pass can be large, so that fluid with relatively large particles can be passed through the housing device 24. The fluid in the flow opening 45 is discharged from the endoscope 10 by suction through the discharge conduit 21.

When an operator or doctor wishes to discontinue the suction, he or she leaves a thumb or finger from the shaft head 56 for a non-depressed state. Then the cap skirt 63 recovers its initial shape by the return force, to move up the cap top portion 62 and the plunger 25. The plunger 25 is switched from the open state to the closed state. The suction channel 17 is disconnected from the discharge conduit 21 as illustrated in Fig. 8A, to discontinue the suction in the distal instrument opening 15.

Similarly, the plunger 25 is set in the open state by depression for suction, and set in the closed state by leaving in a non-depressed state for discontinuing the suction. The tapered valve sleeve 53 has a maximum outer diameter d1 smaller than an inner diameter d2 of the first inner wall 40. The tapered valve sleeve 53 is pressed against the valve engaging surface 42 or inner tapered wall in the closed state, but does not contact the valve engaging surface 42 and the second inner wall 41 in a state other than the closed state. The tapered valve sleeve 53 in the slide is free from frictionally contacting the valve engaging surface 42 and the second inner wall 41 while the plunger 25 slides from a first one of the open and closed positions to a second one of those.

In Figs. 9A and 9B, a comparative example of suction button assembly 80 or shut-off valve assembly is illustrated. The suction button assembly 80 includes a cylinder housing 82 or valve guide of a housing device 81 or cylinder device, a straight cylinder passage 83 with an inner wall, and a plunger 84 or piston unit. The straight cylinder passage 83 is formed in the cylinder housing 82. The plunger 84 is mounted in the straight cylinder passage 83 in a slidable manner. A flow channel 87 is formed through the plunger 84. A valve opening 85 and a flow port 86 are formed at ends of the flow channel 87. Elements similar to those in the suction button assembly 18 of the invention and according to Fig. 10 are designated with identical reference numerals.

A flow opening 88 for a negative pressure source is formed in an inner wall of the straight cylinder passage 83. While the plunger 84 is not depressed in the suction button assembly 80, the valve opening 85 is offset upwards from the flow opening 88 in the plunger longitudinal direction. However, the flow opening 88 cannot be closed completely with an outer wall of the plunger 84, because a diameter of the plunger 84 is smaller than an inner diameter of the straight cylinder passage 83. Should pressure of injecting washing water be high in the injection through the proximal instrument opening 16, the washing water is likely to flow back through the flow channel 87 to leak from the valve opening 85 through the flow opening 88. A seal such as an O-ring is required on an outer wall of the plunger 84 at a portion S (indicated by the phantom line) between the valve opening 85 and the flow opening 88. Considerable resistance is created by frictional contact of the seal with the inner wall of the straight cylinder passage 83. As a result, failure occurs in movement of the plunger 84 to the closed position upon leaving the plunger 84 in a non-depressed state.

In contrast with the suction button assembly 80, the suction button assembly 18 of the invention has the tapered valve sleeve 53 pressed to the valve engaging surface 42 to close the flow opening 45. It is unnecessary to use an O-ring or the like around the plunger 25. The tapered valve sleeve 53 does not cause frictional contact with the second inner wall 41 and the valve engaging surface 42 upon slide of the plunger 25, as described above. Thus, occurrence of frictional contact can be suppressed between the plunger 25 and the inner surface of the housing device 24 even if a flow channel width for fluid is enlarged with the flow channel 60 within the plunger 25.

In the above embodiments, the plunger 25 is kept in the closed state by the return force of the skirt 63 of the cap device 26. However, other structures can be used for keeping the plunger 25 in the closed state, such as a compression coil spring disposed between the cap top portion 62 and the cylinder housing 30 for biasing the plunger 25.

In the above embodiments, the support sleeve 29 is separate from the cylinder housing 30 to constitute the housing device 24. However, the support sleeve 29 may be formed with the cylinder housing 30 by way of a single housing device.

In the above embodiments, the endoscope 10 with the suction button assembly 18 is a bronchoscope. However, the endoscope 10 of the invention may be an endoscope of other type, such as a colonoscope.

In the above cap device, the through opening 65 is formed in the cap top portion 62 for receiving entry of the plunger 25. However, the cap top portion 62 may not have a through opening. Only a center recess can be formed in a lower wall of the cap top portion 62 for receiving entry of the upper end of the plunger 25.

It is possible with the feature of in the invention to combine various known structures of relevant fields of the catheter, the valve, and the like.

Although the present invention has been fully described by way of the preferred embodiments thereof with reference to the accompanying drawings, various changes and modifications will be apparent to those having skill in this field. Therefore, unless otherwise these changes and modifications depart from the scope of the present invention, they should be construed as included therein.

## Claims

1. A suction button assembly for an endoscope including an operation unit (12), a section of an elongated tube (11) for entry in a body cavity, and a suction channel (17) and a discharge conduit (21) disposed with said operation unit, wherein a distal opening is formed in a distal end of said elongated tube, said suction channel is disposed to extend to said distal opening, said discharge conduit is connected to a negative pressure source, said suction button assembly comprising:
a cylinder housing (30), disposed on said operation unit, and including a cylinder passage (40-42), a valve engaging surface (42) and a flow opening (45), said cylinder passage being formed through said cylinder housing, said valve engaging surface being disposed in said cylinder passage, said flow opening formed in a wall of said cylinder passage, said cylinder passage having first and second cylinder openings, said first cylinder opening (32) being connected to said suction channel, said valve engaging surface being tapered with an inner diameter decreasing from said first cylinder opening toward said second cylinder opening (35), said discharge conduit extending from said flow opening;
a plunger (25), contained in said cylinder passage in a slidable manner, and including a stem end (52a), a valve head (53) and a flow channel (60), said stem end protruding from said second cylinder opening, said valve head being movable to and from said valve engaging surface for contact, said flow channel being opposed to said flow opening, wherein said plunger is in a closed position when said stem end is free without being depressed, and is in an open position when said stem end is depressed, and said valve head, when said plunger is in said closed position, contacts said valve engaging surface for interruption between said suction channel and said discharge conduit, and when said plunger is in said open position, leaves from said valve engaging surface for communication between said suction channel and said discharge conduit in cooperation with said flow channel aligned with said flow opening.

2. A suction button assembly as defined in claim 1, wherein said cylinder passage further includes:
a lower inner wall, disposed between said valve engaging surface and said first cylinder opening, and connected in series with said suction channel;
an upper inner wall, disposed between said valve engaging surface and said second cylinder opening, having a smaller inner diameter than said lower inner wall;
wherein a maximum outer diameter of said valve head is smaller than an inner diameter of said lower inner wall, and said upper inner wall contains said stem end in a slidable manner.

3. A suction button assembly as defined in claim 2, further comprising a biasing device for biasing said plunger toward said closed position;
wherein said plunger in said closed position is set in said open position by depressing said stem end into said cylinder passage against said biasing device.

4. A suction button assembly as defined in claim 3, wherein said biasing device is a cap device, secured to said stem end and said cylinder housing, disposed to cover a periphery of said stem end, and formed from resilient material.

5. A suction button assembly as defined in claim 4, wherein said cap device is one piece, and includes a cap top portion secured to said stem end, and a cap skirt formed with said cap top portion, having a sleeve shape, disposed around said stem end, and secured to said cylinder housing;
when said stem end is depressed into said cylinder passage, said cap top portion prevents said stem end from moving down further by contacting said cylinder housing.

6. A suction button assembly as defined in claim 4, further comprising a vent channel formed in said cylinder housing to extend to said discharge conduit;
said vent channel, when said plunger is in said closed position, operates for venting of said discharge conduit, and when said plunger is in said open position, is closed by said cap device.

7. A suction button assembly as defined in claim 1, wherein said discharge conduit extends in a direction perpendicular to a form of said cylinder passage.

8. A suction button assembly as defined in claim 1, wherein said flow channel is a channel, formed through said plunger, and having a flow port and a valve opening, said flow port being formed in a lower stem end of said plunger opposite to said stem end, said valve opening being formed in a wall of said plunger;
said valve opening is positioned in said cylinder passage, and opposed to said flow opening when said plunger is in said open position.

9. A suction button assembly as defined in claim 8, wherein said valve head is disposed at said lower stem end;
said flow channel has an inner diameter increasing toward said flow port in said valve head.

10. A suction button assembly as defined in claim 8, further comprising a regulating device for preventing said plunger from rotating about an axis of said cylinder housing, to keep said valve opening aligned with said flow opening.
